# EUROPEAN PATENT SPECIFICATION

(11) **EP 3 984 987 B1**
(45) Date of publication and mention of the grant of the patent: **11.12.2024**
(21) Application number: 19932874.1
(22) Date of filing: 29.08.2019
(51) Int. Cl.: C07C 45/77, C07C 49/92

(54) **DRY-METHOD SYNTHESIS OF ZINC ACETYLACETONATE UNDER NORMAL PRESSURE**
TROCKENMETHODENSYNTHESE VON ZINKACETYLACETONAT UNTER NORMALDRUCK
PROCÉDÉ DE SYNTHÈSE PAR VOIE SÈCHE D'ACÉTYLACÉTONATE DE ZINC SOUS PRESSION NORMALE

(30) Priority: 13.06.2019 CN 201910509992
(43) Date of publication of application: 20.04.2022
(73) Proprietor: Shenzhen Aimsea Industrial Co., Ltd, Shenzhen, Guangdong 518118 (CN)
(72) Inventor: YAN, Yifeng, Shenzhen, Guangdong 518118 (CN); ZHOU, Zhixin, Shenzhen, Guangdong 518118 (CN); YAN, Qing, Shenzhen, Guangdong 518118 (CN)
(74) Representative: KIPA AB
(86) International application number: PCT/CN2019/103166
(87) International publication number: WO 2020/248382

(56) References cited:
- WO-A1-99/46229
- WO-A1-99/46229
- CN-A- 108 299 175
- CN-A- 109 734 572
- CN-A- 110 105 184
- GB-A- 999 234
- GB-A- 999 234
- JP-A- 2003 342 222

## Description

### FIELD OF THE INVENTION

The present application relates to the technical field of zinc acetylacetonate preparation, in particular to a method for dry synthesizing zinc acetylacetonate under a normal pressure.

### BACKGROUND

Polyvinyl chloride (PVC), polyethylene, polypropylene, polystyrene and ABS are together called as five major thermoplastic synthetic resins. Among a plurality of major general synthetic resins, a yield of the PVC is only lower than that of the polyethylene, occupying a second position. The PVC is formed by polymerizing vinyl chloride, having a plurality of advantages including an excellent chemical corrosion resistance, electrical insulation, flame retardance and mechanical property, as well as a plurality of advantages of a light mass, a high strength, a low cost and more, which has been widely applied to producing a plurality of various plastic products. However, a PVC material is easy to generate a thermal degradation during processing and using, causing a deepened color, and a plurality of reduced various performances, thus a stabilizer must be added to prevent or alleviate the thermal degradation of the PVC material during processing and using. The heat stabilizer is wide in variety, mainly comprising 5 major classes including lead salt, metal soap, organotin compound, organic stabilizer and composite stabilizer, wherein a beta-diketo substance has a low toxicity, being able to improve an initial coloring performance of the PVC remarkably, and extend a heat stability period thereof, which is an auxiliary heat stabilizer with a promising development prospect. Acetylacetone is a simplest beta-diketo, while a complex product of the acetylacetone and zinc, acetylacetone zinc, is a heat stabilizer with an excellent performance, an environmental protection and a high efficiency, has been widely applied to a plurality of industries, in particular to a stabilizer, a heat stabilizer and an antioxidant of a PVC plastic.

Currently, preparation of the zinc acetylacetonate may be achieved by a direct reaction between zinc oxide and the acetylacetone after being dispersed in an organic solvent, may also be achieved through a substitution reaction between an acetylacetone metal compound and the zinc oxide. However, both methods shall be carried out after a dispersion in the organic solvent, having a plurality of complicated steps and processes, while discharging a large amount of industrial wastewater, unfavorable to environment.

WO 99/46229 discloses a method for the preparation of alkaline-earth metal or zinc salts of beta-dicarbonyl compounds in the absence of solvent, wherein formed water is distilled off by vacuum.

GB 999234 discloses a process for the preparation of zinc chelates of diketones by reacting zinc carbonate with a beta-diketone under anhydrous conditions.

Therefore, the current technology needs to be improved and developed.

### BRIEF SUMMARY OF THE DISCLOSURE

According to the defects in the prior art described above, the present application aims to provide a method for dry synthesizing zinc acetylacetonate under the normal pressure, in order to solve a plurality of problems in the prior art during a production process of the zinc acetylacetonate including too much waste water, a larger energy consumption, and a complicated process.

The technical solution of the present application to solve the technical problems is as follows:
a method for dry synthesizing zinc acetylacetonate under a normal pressure, wherein comprising a plurality of following steps:
adding acetylacetone into zinc carbonate hydroxide under a stirring condition, and obtaining a reaction product after a reaction;
dry processing the reaction product to prepare and obtain the zinc acetylacetonate;
wherein the step of adding acetylacetone into zinc carbonate hydroxide under the stirring condition, and obtaining the reaction product after reacting, comprises:

adding acetylacetone drop by drop to the zinc carbonate hydroxide under a first stirring condition;
when an amount of the acetylacetone has half left after being taken out drop by drop, continuing to add the acetylacetone drop by drop to the zinc carbonate hydroxide under a second stirring condition, until the acetylacetone is added drop by drop completely, before preparing and obtaining the reaction product, a second stirring speed is greater than a first stirring speedThe method for dry synthesizing zinc acetylacetonate under the normal pressure, wherein a rotation speed of the first stirring is 200-500rpm.

The method for dry synthesizing zinc acetylacetonate under the normal pressure, wherein a rotation speed of the second stirring is 1000-4000rpm.

The method for dry synthesizing zinc acetylacetonate under the normal pressure, wherein a weight ratio of the alkali type zinc carbonate to the acetylacetone added is 1:1.2-2.

The method for dry synthesizing zinc acetylacetonate under the normal pressure, wherein a temperature of a dry process is 80-120°C.

The method for dry synthesizing zinc acetylacetonate under the normal pressure, wherein a time period of the dry process is 50-70min.

Benefits: the present application provides a method for dry synthesizing zinc acetylacetonate under the normal pressure, by adding acetylacetone into the alkali type zinc carbonate under a stirring condition, and obtaining a reaction product after the reaction, performing the dry process to the reaction product, before obtaining the zinc acetylacetonate. The present application adopts the alkali type zinc carbonate as a zinc source to react with acetylacetone, an activity of the alkali type zinc carbonate is high, being able to perform a fast reaction with the alkali type zinc carbonate under a normal temperature and a normal pressure, while generating carbon dioxide during the reaction process, since a gas of the carbon dioxide generated escapes, making a product concentration be reduced and the reaction be promoted, thus improving a production efficiency of the acetylacetone zinc. Further, the carbon dioxide generated in the reaction process is able to escape from a coating layer of the zinc acetylacetonate formed, leaving a plurality of small holes appearing in a surface of the zinc acetylacetonate, the small holes make the zinc acetylacetonate become loose and fragile, being able to be crushed when being stirred by a stirrer, before exposing an unreacted alkali type zinc carbonate in an inner layer to continue reacting with the acetylacetone, back and forth, until all the alkali type zinc carbonate is completely reacted. A method for synthesizing zinc acetylacetonate provided by the present application is simple to operate, having a small energy consumption, needing no solvent to disperse a reactant during a reaction process. After the reaction is completed, no solvent existing to be treated, no wastewater to be discharged, and the method is clean and environment-friendly.

### BRIEF DESCRIPTION OF THE DRAWINGS

FIG. 1 illustrates a flow chart on a preferred embodiment of dry synthesizing zinc acetylacetonate under a normal pressure in the present application;
FIG. 2 illustrates an NMR spectrogram on a product prepared in an embodiment 3;
FIG. 3 illustrates an NMR spectrogram on a standard sample of zinc acetylacetone.

### DETAILED DESCRIPTION OF EMBODIMENTS

The present application provides a method for dry synthesizing zinc acetylacetonate under a normal pressure, in order to make the purpose, technical solution and the advantages of the present application clearer and more explicit, further detailed descriptions of the present application are stated herein, referencing to the attached drawings and some embodiments of the present application. It should be understood that the detailed embodiments of the application described here are used to explain the present application only, instead of limiting the present application.

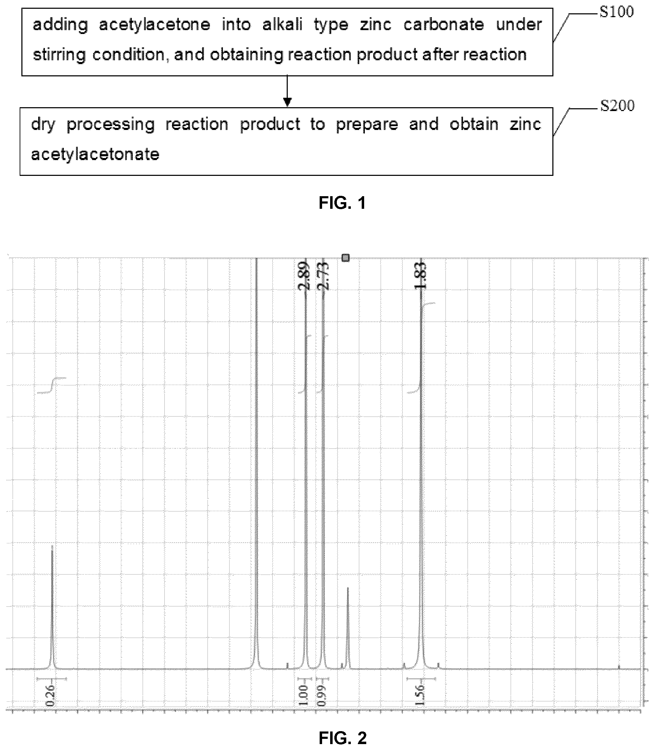

Referencing to FIG.1, which illustrates a flow chart on a preferred embodiment of dry synthesizing zinc acetylacetonate under the normal pressure in the present application. Shown as FIG.1, wherein comprising a plurality of steps:
S100, adding acetylacetone into alkali type zinc carbonate under a stirring condition, and obtaining a reaction product after a reaction;
S200, dry processing the reaction product to prepare and obtain the zinc acetylacetonate.

Specifically, a method for synthesizing zinc acetylacetonate in the prior art, under a dispersion of an organic solvent, reacts zinc oxide with acetylacetone directly, or uses a metal acetylacetone compound and zinc oxide to perform a displacement reaction, both methods need to react under the dispersion of the organic solvent, having a plurality of reaction steps and a complex process, while generating a large amount of industrial wastewater, unfavorable to environment.

The method for dry synthesizing zinc acetylacetonate under the normal pressure adopted by the present embodiment is adding acetylacetone to the alkali type zinc carbonate under a stirring condition, obtaining a reaction product after having a full reaction, while preparing and obtaining the zinc acetylacetonate after performing a drying process to the reaction product. The present embodiment adopts the alkali type zinc carbonate as a zinc source, a reaction equation for the reaction between the alkali type zinc carbonate and the acetylacetone is: 10C₅H₈O₂+3Zn(OH)_{2▪}2ZnCO₃ →5Zn(C₅H₇O₂)₂+8H₂O+2CO₂. Comparing with zinc oxide, a common zinc source in the prior art, the zinc oxide is an amphoteric oxide, being able to react with an acid and a strong base. While the alkali type zinc carbonate is an alkaline, being able to react with a weak acid. The alkali type zinc carbonate is more active than the zinc oxide, easier to have a complex reaction with the acetylacetone; in addition, a density of the zinc oxide is 5.606g·cm⁻³, and a density of the alkali type zinc carbonate is 4.39 g·cm⁻³, the alkali type zinc carbonate is looser than the zinc oxide, having a dispersion and liquidity better.

The present embodiment, wherein the carbon dioxide generated during the reaction between the alkali type zinc carbonate and the acetylacetone may escape from a coating layer of the zinc acetylacetonate formed, leaving a plurality of small holes formed in a surface of the zinc acetylacetonate, the small holes make the zinc acetylacetonate become loose and fragile, being able to be crushed under a stirring by a stirrer, before exposing an unreacted alkali type zinc carbonate in an inner layer to continue react with the acetylacetone, back and forth, until all the alkali type zinc carbonate is completely reacted. The method for synthesizing zinc acetylacetonate provided by the present disclosure needs no solvent or other catalysts to be added, without generating any excess waste water.

In some embodiments, the acetylacetone is added drop by drop to the alkali type zinc carbonate under a stirring condition, before reacting and obtaining a reaction product. In the present embodiment, the acetylacetone is added to the alkali type zinc carbonate in a method of drop by drop, due to a fast reaction speed between the two, if a large amount of acetylacetone is directly poured into the alkali type zinc carbonate, a hard and dense zinc acetylacetonate will be generated quickly, even an escape of the carbon dioxide causes a plurality of pores on the surface, it is still difficult for an agitator to break the zinc acetylacetonate coated on the surface of the alkali type zinc carbonate, thus it will significantly reduce a yield of the zinc acetylacetonate. Instead, adding the acetylacetone drop by drop can make the zinc acetylacetonate produced by the acetylacetone dripped and the alkali type zinc carbonate be broken quickly and mixed in an entire system without covering the alkali type zinc carbonate, also because the acetylacetone is added drop by drop, a concentration of the alkali type zinc carbonate in a whole reaction system is very high, which will also speed up the reaction, making the reaction be able to be carried out at room temperature and a normal pressure, thus improving a yield of the zinc acetylacetonate effectively.

In some embodiments, the acetylacetone is added drop by drop to the alkali type zinc carbonate under a first stirring condition; when an amount of the acetylacetone has half left after being taken out drop by drop, continue to add the acetylacetone drop by drop to the alkali type zinc carbonate under a second stirring condition, until the acetylacetone is added drop by drop completely, before preparing and obtaining the reaction product, a second stirring speed is greater than a first stirring speed. At an initial dripping stage, due to a light weight and looseness of the alkali type zinc carbonate, a too fast rotating speed will cause the alkali type zinc carbonate to float around and affect the reaction with the acetylacetone, thus the first stirring condition having a slower stirring speed is adopted to stir the alkali type zinc carbonate. After over half of the acetylacetone is dripped, due to generating the acetylacetone and a small amount of water, the alkali type zinc carbonate is no longer easy to float around, and a zinc acetylacetonate generated is hard, now it is necessary to adopt a second stirring condition having a faster stirring speed to stir the alkali type zinc carbonate and a small amount of the product, making the zinc acetylacetonate (product) coated on the alkali type zinc carbonate be quickly broken, and exposing an unreacted alkali type zinc carbonate to continue to react with the acetylacetone dripped.

In some embodiments, a rotation speed of the first stirring is 200-500rpm. When the rotation speed of a stirrer is larger than 500rpm, since the alkali type zinc carbonate is mass and loose, an over fast rotation speed will cause the alkali type zinc carbonate floating around, while the rotation speed of the stirrer is less than 200rpm, since the rotation speed is too little, a dispersion of the alkali type zinc carbonate will not be even enough.

In some embodiments, a rotation speed of the second stirring is 1000-4000rpm. After over half acetylacetone is dripped, due to generating the acetylacetone and a small amount of water, the alkali type zinc carbonate is no longer easy to float around, and the zinc acetylacetonate generated is hard, now it is necessary to increase a stirring speed of the stirrer to 1000-4000rpm, making the zinc acetylacetonate covering the alkali type zinc carbonate be crushed quickly, before exposing an unreacted alkali type zinc carbonate to continue reacting with the acetylacetone dripped.

In some embodiments, a weight ratio of the alkali type zinc carbonate to the acetylacetone is 1 :1.2-2. To ensure a higher conversion rate and reduce waste, neither the acetylacetone or the alkali type zinc carbonate shall be excessive too much, since excessive alkali type zinc carbonate will decompose into zinc oxide when being heated, affecting a later stability. While the acetylacetone is volatile, thus taking a slightly over amount than the alkali type zinc carbonate, will increase a conversion rate of the reaction.

In some embodiments, the reaction product is placed in an oven and dried to a constant weight, a temperature of the oven is set as 80-120°C. In the present embodiment, since the acetylacetone and the alkali type zinc carbonate will produce a small amount of water during the reaction, it is necessary to obtain a pure zinc acetylacetonate by a drying process. Preferably, the temperature of the oven is 100°C.

In some embodiments, the reaction product is placed in the oven for the drying process for 50-70 minutes. In the present embodiment, the amount of water generated during the reaction is relatively small, and a 50-70 minutes process in the oven will be able to completely remove the water in the reaction product.

Further detailed descriptions of the present application are stated hereafter, referencing to some embodiments of the present application.

### Embodiment 1

Weigh 100g of acetylacetone and pour into a dropping funnel, then weigh 55g of alkali type zinc carbonate and pour into a stirrer, an initially set of the stirrer is 500rpm, turn on a switch of the dropping funnel, and let the acetylacetone drip down. The reaction proceeds quickly, having carbon dioxide escaping immediately, which will bring up a powder of the alkali type zinc carbonate. Following the dripping of the acetylacetone, more and more zinc acetylacetonate is produced. Increase the rotation speed to 2000rpm, and after 30 minutes, all of the acetylacetone is dripped out, continue stirring at 2000rpm for 20 minutes. After the reaction is over, the product is placed in an oven at 100°C for 1 hour. The zinc content is weighted and measured, having a product yield of 94.56%, and a zinc content of 24.36%.

### Embodiment 2

Weigh 105g of acetylacetone and pour into a dropping funnel, then weigh 56g of alkali type zinc carbonate and pour into a stirrer, an initially set of the stirrer is 300rpm, turn on a switch of the dropping funnel, and let the acetylacetone drip down. The reaction proceeds quickly, having carbon dioxide escaping immediately, which will bring up a powder of the alkali type zinc carbonate. Following the dripping of the acetylacetone, more and more zinc acetylacetonate is produced. Increase the rotation speed to 1500rpm, and after 35 minutes, all of the acetylacetone is dripped out, continue stirring at 2500rpm for 25 minutes. After the reaction is over, the product is placed in an oven at 100°C for 1 hour. The zinc content is weighted and measured, having a product yield of 97.42%, and a zinc content of 25.24%.

### Embodiment 3

Weigh 85g of acetylacetone and pour into a dropping funnel, then weigh 45g of alkali type zinc carbonate and pour into a stirrer, an initially set of the stirrer is 500rpm, turn on a switch of the dropping funnel, and let the acetylacetone drip down. The reaction proceeds quickly, having carbon dioxide escaping immediately, which will bring up a powder of the alkali type zinc carbonate. Following the dripping of the acetylacetone, more and more zinc acetylacetonate is produced. Increase the rotation speed to 3000rpm, and after 30 minutes, all of the acetylacetone is dripped out, continue stirring at 3000rpm for 25 minutes. After the reaction is over, the product is placed in an oven at 100°C for 1 hour. The zinc content is weighted and measured, having a product yield of 96.27%, and a zinc content of 24.98%.

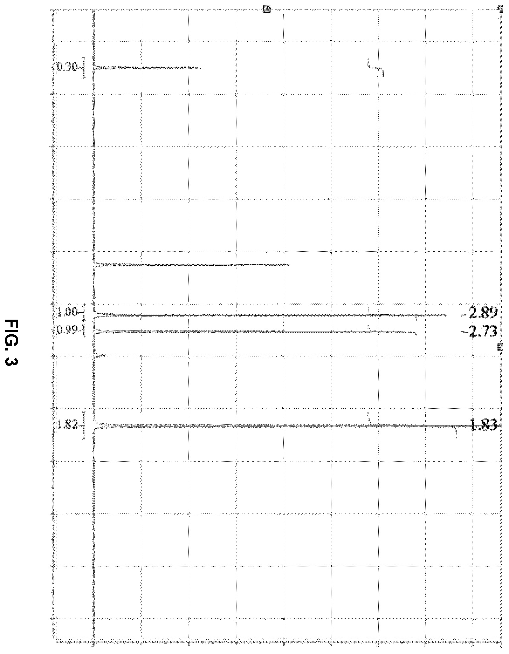

The product prepared in the embodiment 3 and a zinc acetylacetonate standard sample are subjected to an NMR (nuclear magnetic resonance) test respectively, the measured results are shown in FIG.2 and FIG.3. FIG. 2 is an NMR spectrogram on a product prepared in the embodiment 3, wherein 5.23ppm and 1.83ppm reflect an information of the zinc acetylacetonate, an internal standard is DMF, an internal standard is 12.2mg, sample 20.9mg. After a calculation, the content of the zinc acetylacetonate is about 81.6%. FIG.3 is an NMR spectrogram on a standard sample of the zinc acetylacetone. wherein 5.23ppm and 1.83ppm reflect the information of the zinc acetylacetonate, the internal standard is DMF, the internal standard is 12.6mg, sample 22.3mg. After a calculation, the content of the zinc acetylacetonate is about 91.1%. It can be seen from FIG.2 and FIG.3 that the product in the embodiment 3 and the standard sample of the zinc acetylacetone have same characteristic peaks and a same δ value, which fully indicates that a sample synthesized by the present method is zinc acetylacetonate.

All above, the present application provides a method for dry synthesizing zinc acetylacetonate under the normal pressure, by dripping acetylacetone drop by drop into the alkali type zinc carbonate under a stirring condition, and obtaining a reaction product after the reaction completes fully, then placing the reaction product into an oven to dry to a constant weight, before obtaining the zinc acetylacetonate. The present application adopts the alkali type zinc carbonate as a zinc source to react with the acetylacetone, a reaction speed of the alkali type zinc carbonate and the acetylacetone is fast and generating carbon dioxide during the reaction process, since a gas of the carbon dioxide generated escapes, making a product concentration be reduced and the reaction be promoted. Also, the acetylacetone is added drop by drop, making a concentration of the alkali type zinc carbonate in a whole reaction system high, that further promotes the reaction process. Thus the reaction between the alkali type zinc carbonate and the acetylacetone is able to process under a room temperature and a normal pressure, having a simple process. The carbon dioxide generated in the reaction process escapes from a coating layer of the zinc acetylacetonate formed, leaving a plurality of small holes appearing in a surface of the zinc acetylacetonate, the small holes make the zinc acetylacetonate become loose and fragile, being able to be crushed when being stirred by a stirrer, before exposing an unreacted alkali type zinc carbonate in an inner layer to continue reacting with the acetylacetone, back and forth, until all the alkali type zinc carbonate is completely reacted. A common stirrer may be used in the reaction process, having a small energy consumption. Further, during the reaction process, no solvent is required to disperse the reactors, and after the reaction is completed, no solvent existing to be treated, no wastewater to be discharged, and the method is clean and environment-friendly.

It should be understood that, the application of the present application is not limited to the above examples listed, but defined by the appended claims.

## Claims

1. A method for dry synthesizing zinc acetylacetonate under a normal pressure, wherein comprising a plurality of following steps:
adding acetylacetone into zinc carbonate hydroxide under a stirring condition, and obtaining a reaction product after a reaction;
dry processing the reaction product to prepare and obtain the zinc acetylacetonate,
wherein the step of adding acetylacetone into zinc carbonate hydroxide under the stirring condition, and obtaining the reaction product after reacting, comprises:
adding acetylacetone drop by drop to the zinc carbonate hydroxide under a first stirring condition;
when an amount of the acetylacetone has half left after being taken out drop by drop, continuing to add the acetylacetone drop by drop to the zinc carbonate hydroxide under a second stirring condition, until the acetylacetone is added drop by drop completely, before preparing and obtaining the reaction product, a second stirring speed is greater than a first stirring speed.

2. The method for dry synthesizing zinc acetylacetonate under the normal pressure according to claim 1, wherein a rotation speed of the first stirring is 200-500rpm.

3. The method for dry synthesizing zinc acetylacetonate under the normal pressure according to claim 1, wherein a rotation speed of the second stirring is 1000-4000rpm.

4. The method for dry synthesizing zinc acetylacetonate under the normal pressure according to any one of claims 1-3, wherein a weight ratio of the alkali type zinc carbonate to the acetylacetone added is 1:1.2-2.

5. The method for dry synthesizing zinc acetylacetonate under the normal pressure according to claim 1, wherein a temperature of a dry process is 80-120°C.

6. The method for dry synthesizing zinc acetylacetonate under the normal pressure according to claim 1, wherein a time period of a dry process is 50-70min.

## Patentansprüche

1. Verfahren zur Trockensynthese von Zinkacetylacetonat unter Normaldruck, umfassend eine Vielzahl der folgenden Schritte:
Zugabe von Acetylaceton zu Zinkcarbonathydroxid unter Rührbedingungen und der Erhalt eines Reaktionsprodukts nach einer Reaktion;
Trockenverarbeitung des Reaktionsprodukts, um das Zinkacetylacetonat herzustellen und zu erhalten,
wobei der Schritt der Zugabe von Acetylaceton zu Zinkcarbonathydroxid unter Rührbedingungen und der Erhalt des Reaktionsprodukts nach einer Reaktion umfasst:
tropfenweise Zugabe von Acetylaceton zu dem Zinkcarbonathydroxid unter einer ersten Rührbedingung;
wenn eine Menge des Acetylacetons zur Hälfte übrig ist, nachdem es tropfenweise entnommen wurde, das Acetylaceton weiterhin tropfenweise zu dem Zinkcarbonathydroxid unter einer zweiten Rührbedingung zugegeben wird, bis das Acetylaceton tropfenweise vollständig zugegeben ist, bevor das Reaktionsprodukt hergestellt und erhalten wird, eine zweite Rührgeschwindigkeit größer ist als eine erste Rührgeschwindigkeit.

2. Verfahren zur Trockensynthese von Zinkacetylacetonat unter Normaldruck nach Anspruch 1, wobei eine Rotationsgeschwindigkeit des ersten Rührers 200-500 U/min beträgt.

3. Verfahren zur Trockensynthese von Zinkacetylacetonat unter Normaldruck nach Anspruch 1, wobei eine Rotationsgeschwindigkeit des zweiten Rührers 1000-4000 U/min beträgt.

4. Verfahren zur Trockensynthese von Zinkacetylacetonat unter Normaldruck nach einem der Ansprüche 1 bis 3, wobei ein Gewichtsverhältnis von Zinkcarbonat vom Alkalityp zu dem zugesetzten Acetylaceton 1:1,2 bis 2 beträgt.

5. Verfahren zur Trockensynthese von Zinkacetylacetonat unter Normaldruck nach Anspruch 1, wobei eine Temperatur eines Trockenprozesses 80-120 °C beträgt.

6. Verfahren zur Trockensynthese von Zinkacetylacetonat unter Normaldruck nach Anspruch 1, wobei eine Zeitdauer eines Trockenprozesses 50-70 Minuten beträgt.

## Revendications

1. Procédé de synthèse par voie sèche de l'acétylacétonate de zinc sous une pression normale, dans lequel il comprend une pluralité d'étapes suivantes consistant à :
ajouter de l'acétylacétone à de l'hydroxyde de carbonate de zinc dans une condition d'agitation, et obtenir un produit de réaction après une réaction ;
traiter à sec le produit de réaction afin de préparer et d'obtenir l'acétylacétonate de zinc,
dans lequel l'étape consistant à ajouter de l'acétylacétone à de l'hydroxyde de carbonate de zinc dans la condition d'agitation, et à obtenir le produit de réaction après la réaction, comprend :
l'ajout goutte à goutte de l'acétylacétone à l'hydroxyde de carbonate de zinc dans une première condition d'agitation ;
lorsqu'il reste une moitié d'une quantité d'acétylacétone après avoir été retirée goutte à goutte, la poursuite de l'ajout goutte à goutte de l'acétylacétone à l'hydroxyde de carbonate de zinc dans une seconde condition d'agitation, jusqu'à ce que l'acétylacétone soit complètement ajoutée goutte à goutte, avant de préparer et d'obtenir le produit de la réaction, une seconde vitesse d'agitation est supérieure à une première vitesse d'agitation.

2. Procédé de synthèse par voie sèche de l'acétylacétonate de zinc sous la pression normale selon la revendication 1, dans lequel une vitesse de rotation de la première agitation est de 200 à 500 tr/min.

3. Procédé de synthèse par voie sèche de l'acétylacétonate de zinc sous la pression normale selon la revendication 1, dans lequel une vitesse de rotation de la seconde agitation est de 1.000 à 4.000 tr/min.

4. Procédé de synthèse par voie sèche de l'acétylacétonate de zinc sous la pression normale selon l'une quelconque des revendications 1 à 3, dans lequel un rapport de poids entre le carbonate de zinc de type alcalin et l'acétylacétone ajoutée est de 1:1,2 à 2.

5. Procédé de synthèse par voie sèche de l'acétylacétonate de zinc sous la pression normale selon la revendication 1, dans lequel une température d'un traitement à sec est de 80 à 120 °C.

6. Procédé de synthèse par voie sèche de l'acétylacétonate de zinc sous la pression normale selon la revendication 1, dans lequel une durée d'un traitement à sec est de 50 à 70 min.
